# EUROPEAN PATENT APPLICATION

(11) **EP 4 019 553 A1**
(43) Date of publication of application: **29.06.2022**
(21) Application number: 20855429.5
(22) Date of filing: 20.08.2020
(51) Int. Cl.: C08B 30/00, C08J 3/16, A61Q 1/00, A61K 8/02, A61K 8/04, A61K 8/19, A61K 8/60, A61K 8/73

(54) **PARTICLES CONTAINING STARCH, METHOD FOR PRODUCING SAME, AND COSMETIC PREPARATION**

(30) Priority: 20.08.2019 JP 2019150611; 27.09.2019 JP 2019176387
(71) Applicant: JGC Catalysts and Chemicals Ltd., Kawasaki-shi, Kanagawa 212-0013 (JP)
(72) Inventor: ENOMOTO, Naoyuki, Kitakyushu-shi, Fukuoka 808-0027 (JP); WATANABE, Satoshi, Kitakyushu-shi, Fukuoka 808-0027 (JP); SADOWARA, Koki, Kitakyushu-shi, Fukuoka 808-0027 (JP); YANASE, Masashi, Kitakyushu-shi, Fukuoka 808-0027 (JP)
(74) Representative: Dennemeyer & Associates S.A.
(86) International application number: PCT/JP2020/031432
(87) International publication number: WO 2021/033742

(57) **Abstract**

Particles having excellent touch properties are achieved with a water-soluble material having excellent biodegradability. Particles according to the present invention contain a starch having an amylopectin content of 90% by weight or more. The average particle diameter d₁ of the particles is 0.5 to 20 µm, and the maximum particle diameter d₂ is less than 30 µm while being within 3.0 times the average particle diameter.

## Description

### TECHNICAL FIELD

The present invention relates to particles containing a starch having good biodegradability, a production method of the particles, and cosmetics.

### BACKGROUND ART

Petroleum-derived synthetic polymers (plastics) are presently used in various industries. Synthetic polymers are often developed in quest of long-term stability and do not degrade in natural environment. This has caused various environmental problems. For example, plastic products discharged into aqueous environment accumulate for an extended time, which has seriously influenced the ecosystem in oceans and lakes. Also, a recent serious problem is microplastics having a length ranging from 5 mm or less to nm levels. Examples of the microplastics include fine particles contained in cosmetics and the like, small chunks of plastic resins before processed, and large products which have become finer while floating in the sea.

In recent years, several hundred µm-class plastic particles (for example, polyethylene particles) have been formulated in cosmetics in order to enhance touch properties of cosmetics. Plastic particles, which have a low true specific gravity, are difficult to be removed in sewage treatment plants and easy to flow out into rivers, oceans, ponds, and the like. Furthermore, plastic particles, which easily adsorb chemical substances such as pesticides, can affect the human body by bioconcentration. This issue is also pointed out in the United Nations Environment Programme or the like, and countries and various industry groups are considering regulations.

Also, natural cosmetics and organic cosmetics are of increasing interest. The guideline on marking of natural and organic indices for cosmetics (ISO16128) has been established. According to this guideline, raw materials in products are classified into, for example, natural raw materials, naturally derived raw materials, and non-natural raw materials. Based on the content of each raw material, the index is calculated. In the future, indices will be marked on products in accordance with this guideline. Therefore, naturally derived raw materials and furthermore natural raw materials are expected to be required.

Under such circumstances, biodegradable plastics are attracting an attention. The biodegradable plastics are decomposed into water and carbon dioxide by, for example, microorganisms in a natural environment. So, the biodegradable plastics are incorporated in a natural carbon cycle. Especially, cellulose particles being a plant-derived natural raw material do not float on water even when discharged into the environment and also have good biodegradability. Therefore, there is little concern about the possibility that cellulose particles may cause environmental problems. For example, it is known that spherical regenerated cellulose particles of 9 to 400 nm are obtained by neutralizing with an acid a cuprammonium solution in which cellulose is dissolved (for example, see JP-T-2008-84854). It is also known that spherical regenerated cellulose particles are obtained by spraying a cellulose solution to form droplets in a gas phase and bringing the droplets into contact with a coagulation liquid (for example, see JP-A-2013-133355). In these methods, cellulose particles are prepared with celluloses having a type II crystal form obtained through a process of performing intentional chemical modification. Such regenerated cellulose particles are categorized as a naturally derived raw material according to the above-described guideline. On the other hand, powdery cellulose particles having a strength and collapsibility suitable for a scrub agent, which are prepared with celluloses obtained through a process of performing no intentional chemical modification, are also known (for example, see JP-A-2017-88873). Also, it is known that porous-cellulose particles having a type I crystalline form are prepared by granulating and drying celluloses dispersed in an organic solvent by a spray dry method (for example, see JP-A-2-84401).

### SUMMARY OF THE INVENTION

### PROBLEMS TO BE SOLVED BY THE INVENTION

European Chemicals Agency has presented, in "Note on substance identification and the potential scope of a restriction on uses of 'microplastics' Version 1.1" issued on October 16, 2018, the opinion that microplastics are premised on being a synthetic polymer, and naturally derived cellulose, starch, and the like, which have biodegradability, should not be regarded as microplastics.

However, the cellulose according to patent documents is a trans-type polysaccharide having as a constitutional unit a glucose molecule and does not dissolve in water. Therefore, it is considered that degradability in nature of cellulose is not high.

Therefore, an object of the present invention is to achieve particles having excellent touch properties with a water-soluble material having excellent biodegradability.

### SOLUTION TO PROBLEMS

Particles according to the present invention are starch-containing particles having an average particle diameter d₁ of 0.5 to 20 µm and a maximum particle diameter d₂ of less than 30 µm while being less than 3.0 times the average particle diameter. The starch contains 90% by weight or more of amylopectin. Although starch and cellulose are polysaccharides having glucose as a constitutional unit, both are isomers. That is, starch is of cis type, and cellulose is of trans type. This difference has an influence on biodegradability and presence or absence of water solubility. Particles containing water-soluble starch have less concern about causing environmental problems and furthermore have good fluidity. Therefore, these particles can be safely used for uses similar to those of plastic beads. Cosmetics containing such particles can obtain touch properties similar to those of known plastic beads.

Also, the particles may contain, in addition to a starch having an amylopectin content of 90% by weight or more, an inorganic oxide. In that case, it is suitable that the content of the starch be in a range of 30 to 90% by weight, and the content of the inorganic oxide be in a range of 10 to 70% by weight.

On the other hand, the particles may be constituted by a starch having an amylopectin content of 90% by weight or more. The specific surface area of the starch particles is preferably 20 m²/g or more. Furthermore, in an aqueous dispersion liquid having a solid content concentration of 50% by weight of the starch particles, a gelatinization onset temperature based on a DSC curve obtained by using a differential scanning calorimeter is preferably 45°C or higher.

Also, any of the above-described particles preferably has a globulin content of 0.10% by weight or less.

Also, the production method of particles containing starch according to the present invention includes: an emulsification step of mixing a dispersion liquid of starch, a surfactant, and a nonaqueous solvent to prepare an emulsified liquid containing emulsified droplets; a dehydration step of dehydrating the emulsified droplets; and a step of separating the nonaqueous solvent dispersion body obtained in the dehydration step into solid and liquid to obtain spherical starch particles as solid matter.

Also, the emulsified liquid obtained in the emulsification step may be cooled to a range of -50 to 0°C thereby to use a frozen emulsified liquid in which water in the emulsified droplets is frozen.

Any of the above-described particles can be formulated to prepare cosmetics, resin compositions, and paint compositions.

### DESCRIPTION OF EMBODIMENTS

The particles according to the present invention contain a starch having an amylopectin content of 90% by weight or more. In general, starch contains amylose and amylopectin. Amylose is a cis-type polysaccharide and has a structure in which glucose molecules are linearly linked. Amylopectin has a structure in which amylose is branched and linked, and has a high molecular weight. The larger the content of amylose, the higher the swelling properties of starch particles. This causes stickiness when starch particles are formulated in cosmetics. Therefore, the content of amylose needs to be less than 10% by weight (that is, the content of amylopectin needs to be 90% by weight or more). The content of amylopectin is preferably 95% by weight or more and most preferably 98% by weight or more. That is, the larger the content of amylopectin is, the more preferable it is. The content of amylopectin can be measured by gel filtration chromatography, iodine colorimetry, dual wavelength spectrophotometry, or the like.

The content of globulin in the particles (starch in the particles) is preferably 0.10% by weight or less. Globulin is a protein and has strong allergen activity. It is said that this globulin is a material responsible for rice allergy which is relatively common to westerners. Therefore, the content of globulin is preferably as small as possible. It is suitably 0.05% by weight or less and further suitably 0.02% by weight or less. The content of globulin can be decreased by performing, to starch kernels (a raw material of starch), a combination of an enzyme treatment, a high-pressure treatment (for example, 100 to 400 Mpa), immersion in an aqueous sodium chloride solution, an aqueous dilute acid solution, or an aqueous alkaline solution, a washing treatment, and the like.

The average particle diameter d₁ of the particles is 0.5 to 20 µm, and the maximum particle diameter d₂ is less than 30 µm while being within 3.0 times the average particle diameter d₁. The average particle diameter d₁ influences touch properties of cosmetics. When less than 0.5 µm, touch properties, such as rolling feel, persistence of rolling feel, and uniform spreadability, significantly decrease. When more than 20 µm, roughness is felt, and soft feel and moist feel decrease. The average particle diameter d₁ is preferably 1 to 15 µm and most suitably 5 to 10 µm. Also, when the maximum particle diameter d₂ is 30 µm or more, roughness is felt, and soft feel and moist feel decrease. When the maximum particle diameter exceeds 3.0 times the average particle diameter, uniform spreadability decreases.

Also, it is preferable that the sphericity of the particles be 0.85 or more, that is, the particles be spherical. When the particles are spherical particles, the rolling properties of cosmetics improve. The sphericity is particularly preferably 0.90 or more. Here, the sphericity was calculated from a photograph of a scanning electron microscope by an image analysis method.

Also, a coefficient of variance (CV) of particles is preferably 50% or less. When the coefficient of variance of particles exceeds 50%, uniform rolling properties may be impaired. The coefficient of variance of particles is preferably 40% or less and particularly preferably 30% or less. It is noted that although the coefficient of variance of particles is suitably as small as possible, particles having a narrow distribution is industrially difficult to obtain. If it is about 3% or more, production can be performed without particular problem.

The specific surface area of the particles is preferably 20 m²/g or more. When the particles are porous, the biodegradation speed can improve. Therefore, it is more preferably 50 m²/g or more.

Starch has the property of being gelatinized when heated in the coexistence of water. This thermal property can be analyzed using a differential scanning calorimeter (DSC). From a DSC curve (a relationship between temperature and calorie change) obtained by the analysis, a gelatinization onset temperature, a gelatinization peak temperature, and a gelatinization end temperature can be read. Since a gelatinization reaction of starch is an endothermic reaction, the gelatinization onset temperature is a temperature at which the calorie starts to decrease (a temperature at which the DSC curve turns downward), the gelatinization peak temperature is a temperature at which the calorie becomes the minimum value, and the gelatinization end temperature is a temperature at which the calorie decreases again after the gelatinization peak. It is noted that even when the gelatinization onset temperature is equal to or higher than the body temperature, sticky feel due to gelatinization of starch is felt with time after application on the skin on rare occasion. It is considered that this is because the gelatinization onset temperature is not accurately captured. The raw material of starch is primarily a natural material that is plant-derived starch kernels, and there is significant heterogeneity among individual particles of starch kernels (for example, Starch Chemistry, Vol. 32, No. 1, pp. 65 to 83). Therefore, there is a risk that the gelatinization onset temperature may not be measured accurately. Using an ultra-highly sensitive DSC having a detection sensitivity of 0.03 µW or less (for example, Chip-DSC100 manufactured by Linseis USA), thermal properties can be more accurately captured.

In an aqueous starch dispersion liquid having a solid content concentration of 50% by weight, prepared with the starch contained in the particles according to the present invention, the gelatinization onset temperature is desirably 45°C or higher. Furthermore, the gelatinization onset temperature is preferably 50°C or higher and particularly preferably 55°C or higher. Also, the gelatinization peak temperature is preferably 65°C or higher, and the gelatinization end temperature is preferably 75°C or higher. Furthermore, the gelatinization peak temperature is preferably 70°C or higher and particularly preferably 75°C or higher. The gelatinization end temperature is preferably 80°C or higher and particularly preferably 85°C or higher.

Also, when the particles swell in the production step of cosmetics and the like, there is a risk that originally assumed functions may not be obtained. Therefore, it is desirable that the average particle diameter not change during the production step. A test was performed by dispersing the particles according to the present invention in distilled water and applying ultrasonic waves for 60 minutes using an ultrasonic disperser. A ratio (d₃/d₁) between an average particle diameter d₃ after the test and an average particle diameter d₁ before the test is preferably 0.95 to 1.05. When this ratio is less than 0.95, the particles are low in strength, and there is a risk that the particles may collapse due to mechanical loads during the production step, and touch improvement effects may not be obtained. On the other hand, when this ratio exceeds 1.05, the particles swell in water, are likely to be thickened after the production step, and cannot ensure quality stability. When the content of amylopectin is low, and the content of amylose is high, swelling is likely to occur. This ratio is particularly preferably 0.97 to 1.03.

Also, the particles may have a hollow structure in which a cavity is formed inside a shell. Here, the shell is porous. Since such hollow particles are lighter than solid particles having an identical diameter, the number of particles contained in the same weight is larger than solid particles. Even in the case of hollow particles, the specific surface area per unit volume calculated by a BET method is less than 60 m²/cm³. Furthermore, a ratio (T/OD) between a thickness T of the shell and an outer diameter OD of the starch particles is preferably in a range of 0.02 to 0.45. When this ratio exceeds 0.45, the particles are substantially equivalent to solid particles. On the other hand, when this ratio is less than 0.02, the particles are likely to collapse. This ratio is particularly preferably in a range of 0.04 to 0.30. Also, the true specific gravity is preferably in a range of 0.30 to 1.60.

The raw material of the starch contained in the particles is primarily plant-derived starch kernels and inexpensively available. The content of amylopectin in starch kernels differs depending on plant species and breed. For example, the content of amylopectin in corn, potato, wheat, tapioca, and the like is 73 to 83% by weight, and the content of amylopectin in glutinous rice, mochi corn, mochi foxtail millet, and the like is 100% by weight. It is known that the content of amylopectin in a certain species of peas is 0% by weight. As the raw material of the starch, starch kernels having an amylopectin content of 90% by weight or more is suitable. Also, a plurality of species of starch kernels may be mixed to achieve an amylopectin content of 90% by weight or more. Furthermore, this content is preferably 95% by weight or more and most preferably 98% by weight or more.

The shape and size of starch kernels also vary depending on breed. Examples of the shape of starch kernels include polygonal, elliptical, and oval. Starch kernels having any shape can be made spherical by the later-described production method and therefore can be used as the raw material of starch particles. Also, the size of starch kernels is various from 0.5 to 100 µm. For obtaining starch particles having an average particle diameter d₁ of 0.5 to 20 µm, the average particle diameter of starch kernels is suitably less than 20 µm. The average particle diameter of starch kernels is preferably 15 µm or less and most preferably 10 µm or less. Rice starch, which is easily controlled to a small particle diameter, is most preferable.

The gelatinization temperature of starch kernels also varies depending on breed and growing areas. It is known that starch kernels grown in warm regions have a higher gelatinization temperature. The DSC curve of particles can be adjusted by appropriately selecting starch kernels.

Also, suppression of hygroscopicity as well as improvement of dispersibility and fluidity can be achieved by surface-treating the particles. In general, a silicone compound is used as a surface treatment agent. However, awareness of desiliconization has grown in Europe. Therefore, treatments with naturally occurring amino acid, naturally occurring wax components, oil, metal soap, and the like are preferable.

The particles according to the present invention may contain not only the above-described starch but also an inorganic oxide. Particles containing 30 to 90% by weight of the starch and 10 to 70% by weight of the inorganic oxide are hereafter referred to as composite particles. When the content of the inorganic oxide is less than 10% by weight, effects as a binder exhibited by the inorganic oxide decrease, and the number of contact points among starch components increases. This weakens the strength of the particles when gelatinized. On the other hand, when the content of the starch component is less than 30% by weight, soft feel significantly decreases. Notably, it is particularly preferable that the content of the inorganic oxide be in a range of 20 to 50% by weight, and the content of the starch be in a range of 50 to 80% by weight.

As previously described, starch has the property of being gelatinized when heated in the coexistence of water. Gelatinization causes collapse of particles and thickening of a medium. When the starch component is gelatinized in the production step of cosmetics and the like, there is a risk that originally assumed functions may not be obtained. Therefore, it is preferable that even when gelatinization is caused during the production step, the particle shape be maintained, and the medium be not thickened. It is preferable that when an aqueous dispersion liquid (solid content concentration: 10% by weight), in which the composite particles according to the present invention are dispersed, is heated at 80°C for 24 hours, a ratio (V₂/V₁) between a viscosity V₂ of the aqueous dispersion liquid after heating and a viscosity V₁ of the aqueous dispersion liquid before heating be 2.0 or less. When this ratio is 2.0 or more, the particle strength decreases due to gelatinization. Accordingly, there is a risk that the particles may collapse due to heating during the production step, and touch improvement effects may not be obtained.

Examples of the inorganic oxide include a silica component, a titanium oxide component, a magnesium oxide component, an iron oxide component, and a cerium oxide component. Particularly, a silica component is suitable. Examples of a silica component include silicic acid binders and silica particles. Silicic acid binders are obtained by treating an aqueous solution of silicate with cation-exchange resin for dealkalization (for example, removal of Na ions). Examples of silicate include alkali metal silicate such as sodium silicate (water glass) and potassium silicate and silicate of an organic base such as quaternary ammonium silicate.

Silica particles are inorganic oxide particles containing silica. Examples of an inorganic oxide containing silica include composite oxides such as silica-alumina, silica-zirconia, and silica-titania, and silica. The production conditions of the composite particles do not need to be changed corresponding to a difference in the composition of a silica component. Considering that silica particles are formulated in cosmetics, amorphous silica particles are suitable.

It is noted that the average particle diameter d₂ of the silica particles is preferably 5 nm to 1 µm. When the average particle diameter exceeds 1 µm, binder effects decrease, and the dissolution rate of silica in water also decreases. As a result, good biodegradability is sometimes impaired. When the average particle diameter is less than 5 nm, stability as particles is low, which is not preferable in an industrial aspect. Particularly, a range of 10 nm to 0.5 µm is desirable.

Also, the composite particles, constituted by the silica component and the starch component, may contain an inorganic oxide component other than silica, instead of the silica component, if it is 30% or less of the silica component. For example, the composite particles containing 50% by weight of the starch component may contain an inorganic oxide component other than silica, if it is 15% by weight or less. At this time, the silica component comes to 35% by weight or more. When the amount is at this level, the inorganic oxide component can uniformly exist inside the composite particles. An inorganic oxide component other than silica is an inorganic oxide containing at least one of titanium oxide, iron oxide, zinc oxide, magnesium oxide, and cerium oxide. Here, a preferable iron oxide is ferric oxide, α-iron oxyhydroxide, or triiron tetraoxide.

### <Production Method of Particles>

Next, a production method of particles containing a starch having an amylopectin content of 90% by weight or more will be described. First, starch kernels are dispersed in water and subjected to a heating treatment. Accordingly, the starch kernels are solubilized, and a transparent to translucent dispersion liquid of starch is obtained. Subsequently, this dispersion liquid, a surfactant, and a nonaqueous solvent are mixed for emulsification (emulsification step) to obtain an emulsified liquid containing emulsified droplets. In the emulsified droplets, the starch is encapsulated. Next, the emulsified liquid is dehydrated (dehydration step). Accordingly, water in the emulsified droplets is slowly removed. Next, solid and liquid are separated to recover particles as solid matter (solid-liquid separation step). This solid matter is dried and crushed to obtain a powder of particles (drying step).

Hereinafter, each step will be described in detail.

### [Emulsification step]

First, a dispersion liquid of a starch is prepared. The amount of amylopectin contained in starch is 90% by weight or more. Also, the amount of globulin contained in the starch is preferably 0.10% by weight or less. The solid content concentration of the starch is adjusted to a range of 0.01 to 20% by weight, and heating is performed. The heating temperature is preferably 70°C or higher. At lower than 70°C, there is a possibility that gelatinization of starch might not proceed. Also, when the solid content concentration of the dispersion liquid exceeds 20% by weight, viscosity increases, and uniformity of emulsified droplets is impaired. At less than 0.01% by weight, economy deteriorates, and there is no particular advantage. It is noted that the solvent of the dispersion liquid is preferably water.

This dispersion liquid, a nonaqueous solvent, and a surfactant are mixed. The nonaqueous solvent is necessary for emulsification. The nonaqueous solvent is not particularly limited, as long as it is not compatible with water, and a common hydrocarbon solvent can be used. The surfactant is added for forming water-in-oil emulsified droplets. The HLB value of the surfactant is suitably 1 to 10. A most suitable HLB value is selected corresponding to the polarity of the nonaqueous solvent. The HLB value is particularly preferably in a range of 1 to 5. Also, surfactants having different HLB values may be combined.

Next, this mixed solution is emulsified by an emulsification device. At this time, the emulsification conditions are set such that an emulsified liquid containing emulsified droplets having an average diameter of 0.5 to 500 µm is obtained. In the emulsified droplets, the gelatinized starch and water exist. As the emulsification device, a common high-speed shear device can be used. Furthermore, known devices such as a high-pressure emulsification device by which finer emulsified droplets are obtained, a membrane emulsification device by which more uniform emulsified droplets are obtained, and a microchannel emulsification device can be used depending on its intended use.

It is noted that the average diameter of the emulsified droplets was measured as follows. The emulsified liquid is dropped onto a slide glass and covered by a cover glass. A photograph is taken through the cover glass at a magnification of 30 to 2000 times by a digital microscope (VHX-600 manufactured by Keyence Corporation). From the obtained photo projection of the emulsified droplets, 50 droplets are randomly selected. The circle-equivalent diameters of the droplets are calculated by an attached software. The average value of the 50 circle-equivalent diameters was defined as an average diameter (average droplet diameter).

### [Dehydration Step]

Next, the emulsified liquid obtained in the emulsification step is dehydrated. Water is evaporated by heating under normal pressure or reduced pressure. This removes water from the emulsified droplets, and a nonaqueous solvent dispersion body containing particles having a particle diameter of 0.5 to 25 µm is obtained. The starch in the particles contains 90% by weight or more of amylopectin as solid content.

For example, in a thermal dehydration method under normal pressure, a separable flask equipped with a cooling pipe is heated to perform dehydration while recovering a nonaqueous solvent. Also, in a thermal dehydration method under reduced pressure, heating under reduced pressure is performed using a rotary evaporator, an evaporation can, or the like to perform dehydration while recovering a nonaqueous solvent. In the later-described solid-liquid separation step, dehydration is preferably performed until starch particles can be recovered as solid matter from the nonaqueous solvent dispersion body. The moisture content in the nonaqueous solvent dispersion body is preferably 10% by weight or less. With more than this moisture content, the form as spherical particles cannot be maintained in the solid-liquid separation step, and high sphericity cannot be obtained. This moisture content is more preferably 5% by weight or less and most preferably 1% by weight or less. Also, cooling may be performed after dehydration. Amylopectin is crystallized (aged) by cooling thereby to become firm, and the particle strength increases.

### [Solid-Liquid Separation Step]

In the solid-liquid separation step, the solid content is isolated from the nonaqueous solvent dispersion body obtained in the dehydration step, by a known method such as filtration or centrifugation. Accordingly, a cake-like substance of particles is obtained.

Furthermore, the obtained cake-like substance may be washed. This can reduce the surfactant. When the particles are formulated to a liquid formulation such as an emulsion, the surfactant can impair long-term stability. Therefore, the residual amount of the surfactant contained in the particles is preferably 500 ppm or less. For reducing the surfactant, washing with an organic solvent is preferably performed.

### [Drying Step]

In the drying step, heating under normal pressure or reduced pressure is performed to evaporate the nonaqueous solvent contained in the cake-like substance obtained in the solid-liquid separation step. Accordingly, a dried powder of particles having an average particle diameter of 0.5 to 20 µm is obtained.

Also, the dehydration step may be performed after the emulsified liquid obtained in the emulsification step has been cooled at a range of -50 to 0°C. That is, water in the emulsified droplets is frozen to obtain a frozen emulsion. After the frozen emulsion is returned to normal temperature, the dehydration step is performed. When the frozen temperature is -50°C to -10°C, porous particles having a solid structure are obtained. When it is -10 to 0°C, particles having a hollow structure are obtained. At a temperature of about -10 to 0°C, ice crystals gradually grow. As the crystals grow, the starch in the droplets is excluded to the outer circumference of the droplets. Therefore, a cavity is formed inside the shell.

### <Production Method of Composite Particles>

Next, a production method of composite particles will be described. Composite particles can be prepared by adopting a known production method using a spray drying method, an emulsification method, and a coating method. Here, a case in which a silica component is used as the inorganic oxide will be described.

### [Preparation of Starch Dispersion Liquid]

First, starch kernels are dispersed in a solvent and subjected to a heating treatment. Accordingly, the starch kernels are solubilized, and a transparent or translucent dispersion liquid A of a starch is obtained. At this time, starch kernels are selected such that the amount of amylopectin contained in the starch becomes 90% by weight or more. The amount of globulin contained in the starch is preferably 0.10% by weight or less. Also, the solid content concentration of the starch is adjusted to a range of 0.01 to 20% by weight. The heating temperature is preferably 70°C or higher. At lower than 70°C, there is a possibility that gelatinization of the starch might not proceed. Also, when the solid content concentration of the dispersion liquid A exceeds 20% by weight, viscosity increases, and uniformity of the emulsified droplets is impaired. At less than 0.01% by weight, economy deteriorates, and there is no particular advantage. It is noted that the solvent of the dispersion liquid A is preferably water.

### [Preparation of Mixed Dispersion Liquid]

This dispersion liquid A of the starch and a silica component are mixed to prepare a dispersion liquid B. When a silica sol is used as a silica component, a silica sol containing silica-based particles in an amount of 1 to 30% by weight based on solid content is prepared. Also, when silicic acid binders are used as a silica component, silicic acid binders having a solid content concentration of 1.5 to 10.0% by weight are prepared. When the solid content concentration exceeds 10.0% by weight, stability of silicic acid binders deteriorates. This causes generation of gel-like or granular fine silica with time, and sphericity decreases. Particularly, 2.0 to 5.0% by weight is suitable.

### [Granulation]

Next, granulation is performed with the dispersion liquid B by a spray drying method or an emulsification method to obtain composite particles.

### (Spray Drying Method by Spray Dryer)

The dispersion liquid B is sprayed into a hot air stream at a speed of 1 to 3 1/min to prepare composite particles. The temperature of hot air is preferably 70 to 200°C at an inlet and 40 to 60°C at an outlet. When the inlet temperature is lower than 70°C, drying of solid content contained in the dispersion liquid becomes insufficient. Also, when exceeds 200°C, there is a possibility that starch might decompose. Also, when the outlet temperature is lower than 40°C, the drying degree of solid content is low, resulting in the occurrence of adherence to the inside of the device. A more preferable inlet temperature is 100 to 150°C.

### (Emulsification method)

The dispersion liquid B, a nonaqueous solvent, and a surfactant are mixed. Details of the nonaqueous solvent and the surfactant, which have been described in the above-described production method of particles, will be omitted.

This mixed solution is emulsified by an emulsification device (emulsification step). Next, the emulsified liquid is dehydrated (dehydration step). Next, the solid content is isolated from the nonaqueous solvent dispersion body obtained in the dehydration step, by a known method such as filtration or centrifugation (solid-liquid separation step). The nonaqueous solvent contained in the cake-like substance obtained in the solid-liquid separation step is evaporated by heating under normal pressure or reduced pressure (drying step). Accordingly, a dried powder of composite particles having an average particle diameter of 0.5 to 20 µm is obtained. Details of these steps, which are the same as in the previously-described production method of particles, will be omitted.

### (Coating method)

Next, a production method using a coating method will be described.

### [Preparation of Starch Dispersion Liquid]

First, a dispersion liquid of a starch component is prepared. This dispersion liquid contains a starch component in an amount of 5 to 30% by weight based on solid content concentration. This dispersion liquid can be prepared by various methods.

For example, granulation is performed without adding a silica component, by the above-described spray drying method or emulsification method. That is, granulation is performed with the above-described dispersion liquid A. With the thus-obtained starch particles, a dispersion liquid is prepared. At this time, it is suitable that the average particle diameter of the starch particles be 0.5 to 20 µm, and the coefficient of variance (CV value) of particle diameter be 50% or less. Here, the average particle diameter is an average particle diameter based on volume, which is calculated by a laser diffraction scattering method. Also, the sphericity of the starch particles is suitably 0.85 to 1.00. It is noted that although the coefficient of variance (CV value) of particle diameter of the starch particles having a sphericity of 0.85 to 1.00 is desirably less than 5%, starch particles having such a particle size distribution is industrially difficult to obtain. Realistically, the coefficient of variance (CV value) of particle diameter is in a range of 5 to 50%.

Alternatively, a dispersion liquid in which starch kernels are dispersed in a solvent may be used. In this case, starch kernels are coated with a silica component by the later-described method, and granulation is thereafter performed by a spray drying method or an emulsification method. In this manner, desired composite particles are prepared.

With such a dispersion liquid of a starch component (a dispersion liquid of starch particles or a dispersion liquid of starch kernels), coating is performed in the following manner.

### [Coating]

To the dispersion liquid of a starch component, a silicic acid solution is added. Accordingly, a silica component is deposited on the surface of the starch component. At this time, acid or alkali may be added. Accordingly, a silica component is formed on the outermost circumference of the starch component. That is, a silica layer is formed in such a manner as to cover the surface of the starch component as a core. The starch component to serve as a core may be any of starch kernels and starch particles. In this manner, there is obtained a dispersion liquid of composite particles (coated particles) in which the surface of the starch component is covered with a silica layer.

The solid content concentration of the silicic acid component contained in the silicic acid solution is suitably 1 to 40% by weight. When the solid content concentration is low, production efficiency decreases. When the solid content concentration is excessively high, a silica component is deposited in a liquid before deposited on the surface of the starch component, with the result that particles containing only silica are formed. Basically, the thickness of the silica layer is proportional to the amount of the silicic acid component.

While adding the silicic acid solution, it is preferable that the pH be maintained in a range of 8 to 10, and the temperature be maintained in a range of 5 to 80°C. Accordingly, the silica component is densely deposited on the surface of the starch component. Therefore, particles covered with a dense silica layer are obtained. For controlling pH, acid such as hydrochloric acid, nitric acid, sulfuric acid, and organic acid or alkali such as ammonia, organic amine, NaOH, and KOH can be used.

Also, it is preferable that the silicic acid solution be added over 5 to 20 hours. When adding is performed over time, the silica component deposited on the surface becomes dense.

As the silicic acid solution, a solution of silicate such as alkali metal silicate and silicate of an organic base can be used. An example of alkali metal silicate is sodium silicate or potassium silicate, and an example of silicate of an organic base is quaternary ammonium silicate. This silicate solution is preferably dealkalized for use. Particularly preferable is a solution obtained by performing, to a sodium silicate aqueous solution (water glass), a dealkalization treatment (for example, removal of Na ions) with cation-exchange resin. The pH of the solution after the dealkalization treatment is preferably 1 to 8 and more preferably 1.5 to 4.

### [Solid-Liquid Separation]

From the dispersion liquid of the coated particles obtained in this manner, solid content is isolated by a known method such as filtration or centrifugation (solid-liquid separation step). Accordingly, a cake-like substance of the coated particles is obtained. Furthermore, the obtained cake-like substance may be washed. This can reduce impurities such as inorganic salt. When the coated particles are formulated to a liquid formulation such as an emulsion, the inorganic salt can impair long-term stability. Therefore, the residual amount of the inorganic salt contained in the coated particles is preferably 1000 ppm or less. For reducing the inorganic salt, washing with pure water is preferably performed.

### [Drying]

The solvent contained in the cake-like substance obtained in the solid-liquid separation step is evaporated by heating under normal pressure or reduced pressure (drying step) to obtain a dried powder of the coated particles having an average particle diameter of 0.5 to 20 µm. It is preferable that the heating temperature in the drying step be preferably 50 to 150°C, and the heating time be preferably within 24 hours. When the heating temperature is lower than 50°C, a time taken for evaporating the solvent is long, which is not economical. When exceeds 150°C, there is a risk that the starch contained in the coated particles may change in quality, which is not preferable. Also, when the heating time exceeds 24 hours, there is also a risk that the starch contained in the composite particles may change in quality, which is not economical.

It is noted that as necessary, a pulverization step may be provided after drying. The particle diameter distribution of the coated particles can be made in an appropriate range by pulverizing the dry powder of the coated particles. A pulverizer is selected corresponding to a desired particle diameter distribution.

### <Cosmetics>

Cosmetic products can be prepared by formulating the above-described particles and various cosmetic ingredients. According to such cosmetic products, there can be simultaneously obtained a rolling feel, persistence of a rolling feel, and uniform spreadability similar to those of inorganic particles (silica particles) containing a single component and soft feel, and moist feel similar to those of plastic beads. That is, representative texture properties required of a texture improver for cosmetic products can be satisfied.

Specifically, cosmetics are shown in Table 1 according to classifications. Such cosmetics can be manufactured by methods known in the art. The cosmetics are used in various forms such as powders, cakes, pencils, sticks, creams, gels, mousse, liquids, and creams.

Representative categories and components of various cosmetic ingredients are illustrated in Table 2. Furthermore, there may be blended cosmetic ingredients described in the Japanese Standards of Quasi-drug Ingredients 2006 (issued by Yakuji Nippo, Limited, June 16, 2006), International Cosmetic Ingredient Dictionary and Handbook (issued by The Cosmetic, Toiletry, and Fragrance Association, Eleventh Edition, 2006), and the like.

**[Table 1]**

| **Washing cosmetics** | **Soaps. Cleansing foams. Make-up remover creams.** |
|---|---|
| **Skincare cosmetics** | **Moisture retention and skin roughness prevention. Acne. Cuticle care. Massaging. Wrinkle and sag treatments. Dullness and shadow treatments. UV care. Whitening. Antioxidation care.** |
| **Base makeup cosmetics** | **Powder foundations. Liquid foundations. Cream foundations. Mousse foundations. Pressed powders. Makeup bases.** |
| **point makeup cosmetics** | **Eyeshadows. Eyebrow makeup. Eyeliners. Mascaras. Lipsticks.** |
| **Hair-care cosmetics** | **Hair growth. Dandruff prevention. Itch prevention. Conditioning/hair styling. Washing. Perming or waving. Hair coloring or bleaching.** |
| **Body-care cosmetics** | **Washing. Sunscreening. Hand roughness prevention. Slimming. Blood circulation improvement. Itch suppression. Deodorization. Sweat control. Body hair care. Repellents. Body powders.** |
| **Fragrance cosmetics** | **Perfume. Eau de parfum. Eau de toilette. Eau de cologne. Shower cologne. Solid perfume. Body lotion. Bath oil.** |
| **Oral care products** | **Toothpastes. Mouthwashes.** |

**[Table 2]**

| **Ingredients** | **Illustration** |
|---|---|
| **Oils and fats** | **Olive oil. Rapeseed oil. Beef tallow. Jojoba oil.** |
| **Waxes** | **Carnuba wax. Candelilla wax. Beeswax.** |
| **Hydrocarbons** | **Paraffin. Squalane. Synthetic and vegetable squalane. α-olefin oligomers. Microcrystalline** |
| **Fatty acids** | **Stearic acid. Myristic acid. Oleic acid. α-hydroxy acid.** |
| **Alcohols** | **Isostearyl alcohol. Octyldodecanol. Lauryl alcohol. Ethanol. Isopropanol.** |
| **Polyhydric alcohols** | **Ethylene glycol. Triethylene glycol. Polyethylene glycol. Propylene glycol.** |
| | **Glycerin. Diglycerin. 1,3-butylene glycol.** |
| **Es te rs** | **Alkyl glyceryl ethers. Isopropyl myristate. Isopropyl palmitate. Ethyl stearate.** |
| **Saccharides** | **Sorbitol. Glucose. Sucrose. Trehalose. Isomerized sugar combination.** |
| **Silicone oil** | **Methyl polysiloxane. Methyl hydrogen polysiloxane. Methyl phenyl silicone oil.** |
| **Silicone gel** | **Silicone gel crosslinked by silicone-based and/or other organic compounds.** |
| **Surfactants** | **Nonionic. Cationic. Anionic surfactants.** |
| **Fluorine oil** | **Perfluoropolyether** |
| **Various polymers** | **Gum arabic. Carrageenan. Agar. Xanthan gum. Gelatin. Alginic acid. Pllulan. Albumin.** |
| **UV protectors** | **Cinnamic acid such as octyl paramethoxycinnamate. Salicylic acid.** |
| **Inorganic compounds** | **Titanium oxide. Zinc oxide. Aluminum oxide. Aluminum hydroxide. Red iron oxide.** |
| | **Yellow iron oxide. Black iron oxide. Cerium oxide. Zirconium oxide. Silica. Mica. Talc.** |
| **Resin particles** | **Methyl polyacrylate. Nylon. Silicone resin. Silicone rubber. Polyethylene.** |
| **Ingredients having whitening effects** | **Arbutin. Kojic acid. Vitamin C. Linolic acid. Linoleic acid. Lactic acid.** |
| | **Tranexamic acid. Ascorbic acid derivatives (sodium ascorbate, magnesium ascorbate phosphate, ascorbyl dipalmitate, glucoside ascorbate, others). Plant extracts (placenta** |
| **Ingredients having rough skin remedying effects** | **Various vitamins. Carotinoid. Flavonoid. Tannin. Caffeic acid derivatives.** |
| | **Lignan. Saponin. Amino acid. Betaine. Ceramide. Sphingolipid.** |
| | **Retinoic acid and retinoic acid structural analogs. N-acetylglucosamine.** |
| | **ε-aminocaproic acid. α-hydroxy acid. Glycyrrhizic acid. Biopolymers (sodium hyaluronate,** |
| **Other ingredients** | **Antiseptic and preservative agents. Antioxidants. Solvents. Flavors. Water.** |
| | **Modified or unmodified clay minerals. Various organic pigments and dyes.** |

### EXAMPLES

Hereinafter, starch particles formed with a starch having an amylopectin content of 90% by weight or more will be specifically described.

### [Example 1]

In the present example, a MOCHIRU B (registered trademark) manufactured by Joetsu Starch Co., Ltd. was used as starch kernels. That is, 250 g of a MOCHIRU B was added to 4750 g of water, and the obtained suspension liquid was heated and stirred (120°C, 16 hours). Accordingly, a starch dispersion liquid having a solid content concentration of 5% by weight was obtained.

This dispersion liquid, a nonaqueous solvent, and a surfactant are mixed. Here, 40 g of the dispersion liquid was diluted with 160 g of pure water to have a solid content concentration of 1% by weight. This diluted dispersion liquid was added to a mixed solution of 3346 g of heptane (manufactured by Kanto Chemical Co., Inc.) and 25 g of an AO-10V surfactant (manufactured by Kao Corporation). The mixture was stirred using an emulsification disperser (T.K. Robomix manufactured by Primix Corporation) at 10000 rpm for 10 minutes. This initiated emulsification, and an emulsified liquid containing emulsified droplets was obtained. This emulsified liquid was heated at 60°C for 16 hours to dehydrate the emulsified droplets. Furthermore, this dehydrated emulsified liquid was stored under cooling at 2°C for 16 hours and thereafter filtered through a quantitative filter paper (No.2 manufactured by Advantec Toyo Kaisha, Ltd.) using a Buchner funnel (3.2 L manufactured by Sekiya Chemical Glass Apparatus Co., Ltd.). Thereafter, washing with heptane was repeated to remove the surfactant. The thus obtained cake-like substance was dried at 60°C for 12 hours. This dried powder was passed through a 250 mesh sieve (standard sieve for JIS tests) to obtain starch particles.

The preparation conditions of the starch particles are illustrated in Table 3. Also, physical properties of the starch particles (powder) were measured in the following method. The measurement results are illustrated in Table 4 together with the results of other examples.

### (1) Content of amylopectin

The content of amylopectin was measured using an amylose/amylopectin analysis kit (manufactured by Biocon (Japan) Ltd.). In accordance with a measurement procedure prescribed by the kit, the total starch amount and the amylose amount were measured, and "{1 - (amylose amount/total starch amount)} × 100" was defined as the content of amylopectin (% by weight).

### (2) Globulin residual amount

The globulin residual amount was measured by an SDS-polyacrylamide gel electrophoresis method. First, 2 g of sodium dodecyl sulphate (manufactured by FUJIFILM Wako Pure Chemical Corporation), 24 g of urea (manufactured by FUJIFILM Wako Pure Chemical Corporation), 10 g of glycerin, and 0.76 g of tris(hydroxymethyl)aminomethane (manufactured by Tokyo Chemical Industry Co., Ltd.) were dissolved with distilled water. This solution was adjusted to pH 6.8 with a 1 N aqueous hydrochloric acid solution. Furthermore, 2.5 g of 2-mercaptoethanol (manufactured by FUJIFILM Wako Pure Chemical Corporation) was added to obtain 100 mL of a protein extract. The powder (1 g) of the starch particles obtained in the example was added to 0.7 mL of the protein extract. The mixture was stirred and then left to stand for 24 hours. Thereafter, the supernatant liquid was isolated by centrifugation. This supernatant liquid (0.01 mL) was charged to a PGME gel (manufactured by Funakoshi Co., Ltd.) for electrophoresis. Thereafter, this gel was immersed in a CBB staining reagent (manufactured by BioDynamics Laboratory Inc.) for staining. The stained gel was subjected to image analysis (automated determination by a Luminescent image analyzer LAS-100 plus manufactured by Fujifilm Corporation and an Image Gauge manufactured by Fujifilm Corporation)) to calculate a globulin residual amount. This method was used to measure the globulin residual amount of the starch particles obtained in Example 1. The result was 0.20% by weight.

### (3) Average particle diameter, maximum particle diameter, and coefficient of variance of particles for particles

The particle size distribution of the particles was measured by laser diffractometry. A median value was calculated from this particle size distribution and defined as an average particle diameter d₁. Also, a largest particle diameter detected by the particle size distribution was defined as a maximum particle diameter d₂. Furthermore, from the particle size distribution (population), a standard deviation σ and a population mean µ were calculated to obtain a coefficient of variance (CV = σ/µ) of particles. In Table 4, these are indicated in percentage. Here, the particle size distribution was measured using an LA-950v2 manufactured by Horiba, Ltd.

### (4) Average particle diameter ratio based on presence or absence of ultrasonic dispersion

Dispersion was performed using the above-described measuring apparatus (LA-950v2) by setting the dispersion conditions to "ultrasonic for 60 minutes". After this ultrasonic dispersion test, the particle size distribution of the starch particles was measured. The median value in this particle size distribution was defined as an average particle diameter d₃ after ultrasonic dispersion. From this, an average particle diameter ratio (d₃/d₁) before and after the ultrasonic dispersion test was calculated.

### (5) Sphericity

A photo projection is obtained by taking a picture at a magnification of 2000 to 250,000 times through a transmission electron microscope (H-8000 manufactured by Hitachi, Ltd.). From this photo projection, optional 50 particles were selected, and a longest diameter D_{L} and a short diameter Ds orthogonal to the longest diameter of each particle were measured to calculate a ratio (D_{S}/D_{L}). An average value of the ratios was defined as a sphericity.

### (6) Pore volume and pore diameter

The powder (10 g) of the starch particles was put in a crucible and dried at 105°C for 1 hour. Thereafter, the resultant product was poured in a desiccator and cooled to room temperature. Subsequently, 0.15 g of a sample was poured in a washed cell. While vacuum deaeration is performed using a Belsorp-mini II (manufactured by Bell Japan, Inc.), nitrogen gas is adsorbed to this sample. Thereafter, nitrogen is desorbed. From the obtained adsorption isotherm, an average pore diameter is calculated by a BJH method. Also, from the formula "pore volume (ml/g) = (0.001567 × (V - Vc)/W)", a pore volume was calculated. Here, V represents an adsorption amount (ml) in a standard state at a pressure of 735 mmHg. Vc represents a volume (ml) of a cell blank at a pressure of 735 mmHg. W represents a mass (g) of a sample. Also, a density ratio between nitrogen gas and liquid nitrogen was 0.001567.

### (7) Gelatinization onset temperature, gelatinization peak temperature, and gelatinization end temperature

The starch particles and distilled water were mixed to adjust solid content concentration at 50% by weight. This sample (20 mg) was placed in a sealed pressure resistant container, and a DSC curve when heated at a temperature increasing rate of 2°C/min to 120°C was obtained using a differential scanning calorimeter (Thermo-plus-EVO-DSC8230 manufactured by Rigaku Corporation). From this DSC curve, a gelatinization onset temperature (T₁), a gelatinization peak temperature (T₂), and a gelatinization end temperature (T₃) can be read.

### (8) Viscosity ratio based on presence or absence of heating

The powder of the starch particles and distilled water were mixed to adjust the solid content concentration at 10% by weight. This sample (80 g) was placed in a pressure resistance-type sealed container and heated at 80°C for 24 hours. A viscosity V₂ of the aqueous dispersion liquid after heating and a viscosity V₂ of the aqueous dispersion liquid before heating were measured using a viscometer (TVB10-type viscometer manufactured by Toki Sangyo Co., Ltd), and a viscosity ratio (V₂/V₁) was calculated.

### [Example 2]

The dispersion liquid (200 g) having a solid content concentration of 5% by weight prepared in Example 1, without being diluted, was added in a mixed solution of 3346 g of heptane and 25 g of a surfactant (AO-10V). Using an emulsification disperser, this solution was stirred at 10000 rpm for 10 minutes for emulsification. The obtained emulsified liquid was left to stand in a constant temperature bath at -5°C for 72 hours to freeze water in the emulsified droplets. Thereafter, this liquid was increased in temperature to normal temperature for thawing. The resultant product was filtered and washed to remove the surfactant, in a similar manner to Example 1. With the thus obtained cake-like substance, starch particles were prepared in a similar manner to Example 1.

The inside structure of the starch particles obtained in the present example was studied. To about 1 g of epoxy resin (EPO-KWICK manufactured by BUEHLHER), 0.1 g of the powder was uniformly mixed. The mixture was hardened at normal temperature. Thereafter, a sample was prepared using a FIB processor (FB-2100 manufactured by Hitachi, Ltd.). A SEM image of this sample was photographed using a transmission electron microscope (HF-2200 manufactured by Hitachi, Ltd.) under the condition of an acceleration voltage of 200 kV. As a result, this sample was particles having a hollow structure in which a cavity was formed inside a shell. From this SEM image, a thickness T and an outer diameter OD of the shell were measured, and a thickness ratio (T/OD) of the shell was calculated.

### [Example 3]

In a similar manner to Example 2, an emulsified liquid was prepared. This emulsified liquid was left to stand in a freezer at -25°C for 72 hours. Thereafter, starch particles were prepared in a similar manner to Example 2.

### [Example 4]

With a Waxy Starch Y (manufactured by Sanwa Starch Co., Ltd.) as starch kernels, a dispersion liquid having a solid content concentration of 5% by weight was prepared in a similar manner to Example 1. This dispersion liquid was diluted with pure water to have a solid content concentration of 1% by weight. This diluted solution (200 g) was added in a mixed solution of 3346 g of heptane and 25 g of a surfactant (AO-10V). Thereafter, starch particles were prepared in a similar manner to Example 1.

### [Example 5]

In a similar manner to Example 1, a dispersion liquid having a solid content concentration of 5% by weight was prepared. This dispersion liquid (200 g), without being diluted, was added in a mixed solution of 3346 g of heptane and 25 g of a surfactant (AO-10V). Thereafter, starch particles were prepared in a similar manner to Example 1. However, the rotation speed of the emulsification disperser in the emulsification step was changed to 2000 rpm, and the heating time (dehydration time) of the emulsified liquid in the dehydration step was changed to 24 hours.

### [Example 6]

The rotation speed of the emulsification disperser was changed to 5000 rpm, and the heating time of the emulsified liquid was changed to 16 hours. Otherwise, starch particles were prepared in a similar manner to Example 5.

### [Example 7]

The rotation speed of the emulsification disperser was changed to 800 rpm. Otherwise, starch particles were prepared in a similar manner to Example 5.

### [Example 8]

As starch kernels, 125 g of a MOCHIRU B and 125 g of a Fine Snow (registered trademark) manufactured by Joetsu Starch Co., Ltd. were used. Otherwise, starch particles were prepared in a similar manner to Example 4.

### [Example 9]

As starch kernels, 187.5 g of a MOCHIRU B and 62.5 g of a Fine Snow, both manufactured by Joetsu Starch Co., Ltd., were used. Otherwise, starch particles were prepared in a similar manner to Example 4.

### [Example 10]

In the present example, a MOCHIRU B manufactured by Joetsu Starch Co., Ltd. is immersed in alkali and treated with an enzyme to reduce the globulin amount. That is, 1 kg of a MOCHIRU B was added to 4 kg of pure water to prepare a suspension liquid. To this suspension liquid, 5% by weight of an aqueous sodium hydroxide solution was added to adjust the pH to 9.0. To the resultant product, 10 g of an enzyme (Aroase AP-10 manufactured by Yakult Pharmaceutical Industry Co., Ltd.) was added. The mixture was stirred at room temperature for 24 hours. Subsequently, this dispersion liquid was filtered through a quantitative filter paper (No.2 manufactured by Advantec Toyo Kaisha, Ltd.) using a Buchner funnel (3.2 L manufactured by Sekiya Chemical Glass Apparatus Co., Ltd.). Furthermore, washing with pure water was performed to remove the residual enzyme and the decomposed protein. The thus obtained cake-like substance was dried at 60°C for 12 hours. This dried powder was passed through a 250 mesh sieve (standard sieve for JIS tests) to obtain a powder of a starch having a reduced globulin content.

This powder (250 g) was suspended in 4750 g of pure water. This suspension liquid was heated and stirred (120°C, 16 hours) to prepare a starch dispersion liquid having a solid content concentration of 5% by weight. Thereafter, starch particles were prepared in a similar manner to Example 1. The globulin residual amount of the starch particles obtained in the present example was 0.02% by weight.

### [Example 11]

In the present embodiment, 0.5 kg of a MOCHIRU B and 0.5 kg of a Fine Snow, both manufactured by Joetsu Starch Co., Ltd., were added to 4 kg of pure water to prepare a suspension liquid. Thereafter, starch particles were prepared in a similar manner to Example 10. The globulin residual amount of the starch particles obtained in the present example was 0.02% by weight.

### [Comparative Example 1]

A similar operation to Example 4 was performed, except that the dehydration conditions of the emulsified liquid were changed to 45°C for 3 hours. However, the substance obtained by performing filtration and washing after dehydration had a film shape, and particles could not be observed in the observation through an optical microscope. It is considered that dehydration was insufficient, and this caused coalescence of liquid droplets, with the result that particles could not be prepared.

### [Comparative Example 2]

As starch kernels, a Fine Snow manufactured by Joetsu Starch Co., Ltd. was used. Otherwise, preparation of starch particles and measurement of physical properties were performed in a similar manner to Example 4.

### [Comparative Example 3]

In the present comparative example, a dispersion liquid having a solid content concentration of 20% by weight was used instead of the starch dispersion liquid having a solid content concentration of 5% by weight in Example 5. That is, 1000 g of a MOCHIRU B was suspended in 4000 g of pure water to prepare a dispersion liquid having a solid content concentration of 20% by weight. Otherwise, preparation of starch particles and measurement of physical properties were performed in a similar manner to Example 5. However, the rotation speed of the emulsification disperser in the emulsification step was changed to 800 rpm.

**[Table 3]**

| | **Dispersion liquid of starch** | | **Emulsification conditions** | | **Dehydration (or freezing) conditions** | |
|---|---|---|---|---|---|---|
| | **Type of starch** | **Concentration [%]** | **Emulsification dispersion rate [rpm]** | **Emulsification time (min.)** | **Condition** | **Time [Hr.]** |
| **Example 1** | **[1]** | **1.0** | **10000** | **10** | **Heating; 60°C** | **16** |
| **Example 2** | **[1]** | **5.0** | **10000** | **10** | **Freezing; -5°C** | **72** |
| **Example 3** | **[1]** | **5.0** | **10000** | **10** | **Freezing; -25°C** | **72** |
| **Example 4** | **[2]** | **1.0** | **10000** | **10** | **Heating; 60°C** | **16** |
| **Example 5** | **[1]** | **5.0** | **2000** | **10** | **Heating; 60°C** | **24** |
| **Example 6** | **[1]** | **5.0** | **5000** | **10** | **Heating; 60°C** | **16** |
| **Example 7** | **[1]** | **5.0** | **800** | **10** | **Heating; 60°C** | **24** |
| **Example 8** | **[1] + [3]** | **1.0** | **10000** | **10** | **Heating; 60°C** | **16** |
| **Example 9** | **[1] + [3]** | **1.0** | **10000** | **10** | **Heating; 60°C** | **16** |
| **Example 10** | **A** | **1.0** | **10000** | **10** | **Heating; 60°C** | **16** |
| **Example 11** | **B** | **1.0** | **10000** | **10** | **Heating; 60°C** | **16** |
| **Comparative Example 1** | **[2]** | **1.0** | **10000** | **10** | **Heating; 45°C** | **3** |
| **Comparative Example 2** | **[3]** | **1.0** | **10000** | **10** | **Heating; 60°C** | **16** |
| **Comparative Example 3** | **[1]** | **20.0** | **800** | **10** | **Heating; 60°C** | **24** |

| | | | | | | |
|---|---|---|---|---|---|---|
| [1]: MOCHIRU B (amylopectin 100%, derived from glutinous rice) manufactured by Joetsu Starch Co., Ltd. [2]: Waxy Starch Y (amylopectin 100%, derived from corn) manufactured by Sanwa Starch Co., Ltd. [3]: Fine Snow (amylopectin 80%, derived from nonglutinous rice) manufactured by Joetsu Starch Co., Ltd. A: Powder having a globulin residual amount lower than [1] B: Powder having a globulin residual amount lower than "[1] + [3]" | | | | | | |

**[Table 4]**

| | **Starch particles** | | | | | | | | | | | | | |
|---|---|---|---|---|---|---|---|---|---|---|---|---|---|---|
| | **Amylopectin content (%)** | **Average particle diameter (d₁) µm** | **Maximum particle diameter (d₂) µm** | **d₂/d₁** | **Sphericity** | **Coefficient of variance of particles (CV) %** | **Pore volume ml/g** | **d₃/d₁** | **Inside structure** | **T/OD** | **Gelatinization onset temperature (°C)** | **Gelatinization peak temperature (°C)** | **Gelatinization end temperature (°C)** | **Viscosity ratio before and after heating** |
| **Example 1** | **100** | **1.5** | **4.5** | **3.0** | **0.95** | **29** | **0.2** | **1.01** | **Porous solid** | **-** | **95** | **98** | **109** | **1.4** |
| **Example 2** | **100** | **5.5** | **15.2** | **2.8** | **0.90** | **35** | **0.7** | **1.01** | **Porous hollow** | **0.12** | **89** | **94** | **106** | **1.3** |
| **Example 3** | **100** | **6.3** | **15.2** | **2.4** | **0.88** | **34** | **0.7** | **1.01** | **Porous solid** | **-** | **95** | **99** | **105** | **1.3** |
| **Example 4** | **100** | **1.6** | **4.5** | **2.8** | **0.95** | **29** | **0.2** | **1.01** | **Porous solid** | **-** | **92** | **99** | **106** | **1.4** |
| **Example 5** | **100** | **13.2** | **26.1** | **2.0** | **0.88** | **36** | **0.2** | **1.01** | **Porous solid** | **-** | **94** | **99** | **106** | **1.2** |
| **Example 6** | **100** | **9.3** | **19.9** | **2.1** | **0.89** | **37** | **0.2** | **1.01** | **Porous solid** | **-** | **95** | **98** | **107** | **1.2** |
| **Example 7** | **100** | **18.9** | **29.9** | **1.6** | **0.92** | **41** | **0.2** | **1.00** | **Porous solid** | **-** | **94** | **94** | **102** | **1.2** |
| **Example 8** | **90** | **1.9** | **4.5** | **2.4** | **0.91** | **41** | **0.2** | **1.03** | **Porous solid** | **-** | **80** | **92** | **99** | **1.9** |
| **Example 9** | **95** | **1.8** | **4.5** | **2.5** | **0.91** | **39** | **0.2** | **1.02** | **Porous solid** | **-** | **82** | **95** | **100** | **1.8** |
| **Example 10** | **100** | **1.7** | **4.9** | **2.9** | **0.95** | **30** | **0.2** | **1.01** | **Porous solid** | **-** | **92** | **97** | **108** | **1.4** |
| **Example 11** | **90** | **1.8** | **5.1** | **2.8** | **0.95** | **31** | **0.2** | **1.01** | **Porous solid** | **-** | **81** | **90** | **100** | **1.9** |
| **Comparative Example 1** | **100** | **-** | **-** | **-** | **-** | **-** | **-** | **-** | **-** | **-** | **-** | **-** | **-** | **-** |
| **Comparative Example 2** | **80** | **1.1** | **4.5** | **4.1** | **0.91** | **32** | **0.1** | **1.23** | **Porous solid** | **-** | **39** | **53** | **66** | **2.3** |
| **Comparative Example 3** | **100** | **29.0** | **102.3** | **3.5** | **0.85** | **55** | **0.2** | **1.02** | **Porous solid** | **-** | **94** | **98** | **109** | **1.3** |

Next, the composite particles will be specifically described.

### [Example 12]

First, 250 g of a MOCHIRU B manufactured by Joetsu Starch Co., Ltd. was suspended in 4750 g of pure water. This suspension liquid was heated and stirred (120°C, 16 hours) to prepare a starch dispersion liquid A having a solid content concentration of 5% by weight. To this dispersion liquid A, 833 g of a silica sol (Cataloid SI-30) manufactured by JGC Catalysts and Chemicals Ltd. was added to prepare a dispersion liquid B having a solid content concentration of 9% by weight.

This dispersion liquid B, a nonaqueous solvent, and a surfactant are mixed. In the present example, 23 g of the dispersion liquid B was diluted with 177 g of pure water to have a solid content concentration of 1% by weight. This diluted dispersion liquid was added to a mixed solution of 3346 g of heptane manufactured by Kanto Chemical Co., Inc. and 25 g of a surfactant (AO-10V) manufactured by Kao Corporation. The mixture was stirred at 10000 rpm for 10 minutes using an emulsification disperser (T.K. Robomix manufactured by Primix Corporation). This initiated emulsification, and an emulsified liquid containing emulsified droplets was obtained. This emulsified liquid was heated at 60°C for 16 hours to dehydrate the emulsified droplets. Furthermore, the emulsified liquid after dehydration was stored under cooling at 2°C for 16 hours and thereafter filtered through a quantitative filter paper (No.2 manufactured by Advantec Toyo Kaisha, Ltd.) using a Buchner funnel (3.2 L manufactured by Sekiya Chemical Glass Apparatus Co., Ltd.). Thereafter, washing with heptane was repeated to remove the surfactant. The thus obtained cake-like substance was dried at 60°C for 12 hours. This dried powder was passed through a 250 mesh sieve (standard sieve for JIS tests) to obtain a powder of composite particles.

The preparation conditions of the composite particles are illustrated in Table 5. Also, the physical properties of the composite particles (powder) were measured by the method described in Example 1. The results are illustrated in Table 6. The same applies to the later-described Examples and Comparative Examples.

### [Example 13]

With a Waxy Starch Y (manufactured by Sanwa Starch Co., Ltd.) as starch kernels, a dispersion liquid B having a solid content concentration of 9% by weight was prepared in a similar manner to Example 12. This dispersion liquid was diluted with pure water to have a solid content concentration of 1% by weight. This diluted solution (200 g) was added to a mixed solution of 3346 g of heptane and 25 g of a surfactant (AO-10V). Otherwise, the procedure is similar to that of Example 12.

### [Example 14]

In a similar manner to Example 12, a dispersion liquid B having a solid content concentration of 9% by weight was prepared. This dispersion liquid (200 g), without being diluted, was added to a mixed solution of 3346 g of heptane and 25 g of a surfactant (AO-10V). Also, the rotation speed of the emulsification disperser in the emulsification step was changed to 2000 rpm, and the heating time (dehydration time) of the emulsified liquid in the dehydration step was changed to 24 hours. Otherwise, the procedure is similar to that of Example 12.

### [Example 15]

The rotation speed of the emulsification disperser was changed to 5000 rpm, and the heating time of the emulsified liquid was changed to 16 hours. Otherwise, the procedure is similar to that of Example 14.

### [Example 16]

The rotation speed of the emulsification disperser was changed to 800 rpm. Otherwise, the procedure is similar to that of Example 14.

### [Example 17]

As starch kernels, 125 g of a MOCHIRU B and 125 g of a Fine Snow were used. Otherwise, the procedure is similar to that of Example 13.

### [Example 18]

As starch kernels, 187.5 g of a MOCHIRU B and 62.5 g of a Fine Snow were used. Otherwise, the procedure is similar to that of Example 13.

### [Example 19]

The dispersion liquid B (200 g) prepared in Example 12, without being diluted, was added to a mixed solution of 3346 g of heptane and 25 g of a surfactant (AO-10V). Using an emulsification disperser, this solution was stirred at 10000 rpm for 10 minutes for emulsification. The obtained emulsified liquid was left to stand in a freezer at - 25°C for 72 hours to freeze water in the emulsified droplets. Thereafter, the temperature of this liquid was increased to normal temperature for thawing. Otherwise, the procedure is similar to that of Example 12.

### [Example 20]

A dispersion liquid B having a solid content concentration of 8% was prepared with 1250 g of a silica sol (Cataloid SI-550) manufactured by JGC Catalysts and Chemicals Ltd., instead of 833 g of the silica sol used in Example 12. Otherwise, the procedure is similar to that of Example 12.

### [Example 21]

A dispersion liquid B having a solid content concentration of 8% was prepared with 1562 g of a silica sol (SS-160) manufactured by JGC Catalysts and Chemicals Ltd., instead of 833 g of the silica sol used in Example 12. Otherwise, the procedure is similar to that of Example 12.

### [Example 22]

To the dispersion liquid A (5000 g) prepared in Example 12, 278 g of a silica sol (Cataloid SI-30) manufactured by JGC Catalysts and Chemicals Ltd. was added to prepare a dispersion liquid B. That is, the silica component in this dispersion liquid is 25% by weight, and the starch component is 75% by weight. Otherwise, the procedure is similar to that of Example 12.

### [Example 23]

To the dispersion liquid A (5000 g) prepared in Example 12, 147 g of a silica sol (Cataloid SI-30) manufactured by JGC Catalysts and Chemicals Ltd. was added to prepare a dispersion liquid B. That is, the silica component in this dispersion liquid is 15% by weight, and the starch component is 85% by weight. Otherwise, the procedure is similar to that of Example 12.

### [Example 24]

A dispersion liquid B having a solid content concentration of 5% was prepared with 5000 g of a silicic acid solution (solid content concentration: 5% by weight), instead of the silica sol used in Example 12. Otherwise, the procedure is similar to that of Example 12.

### [Example 25]

In the present example, a coating method was used to prepare composite particles. First, 9.0 kg of pure water is added to 1.0 kg of starch particles (MOCHIRU B) manufactured by Joetsu Starch Co., Ltd to obtain a dispersion liquid A of starch particles having a solid content concentration of 10.0% by weight. The average particle diameter of the starch particles was calculated from a particle size distribution measured by laser diffractometry. Here, the particle size distribution of the starch particles was measured using an LA-950v2 (manufactured by Horiba, Ltd.), and the volume-converted average particle diameter (D₁) was calculated. The average particle diameter (D₁) of the obtained starch particles was 6.8 µm. Also, the coefficient of variance (CV value) of particles was 31%, and the sphericity was 0.85. The obtained starch particles were used as nucleus particles.

Next, a sodium silicate aqueous solution was subjected to cation exchange to prepare a silicic acid solution (concentration of a silica component: 4.5% by weight). This silicic acid solution (3.9 kg) was added to the dispersion liquid A over 16 hours. While added, the pH was maintained at 9.0, and the solution temperature was maintained at 40°C. As alkali in maintaining pH, 1 kg of ammonia water (concentration: 15% by weight) was used. After the silicic acid solution was added, a Buchner funnel (3.2 L manufactured by Sekiya Chemical Glass Apparatus Co., Ltd.) was used to perform filtration through a quantitative filter paper (No.2 manufactured by Advantec Toyo Kaisha, Ltd.). Thereafter, washing with pure water was repeated, and the obtained cake-like substance was dried at 80°C for 16 hours. This dried powder was passed through a 250 mesh sieve (standard sieve for JIS tests) to obtain a powder of composite particles. The measurement results of the physical properties of this powder are illustrated in Table 4. In the composite particles obtained in the present example, the surface of the starch particles was coated with a silica layer. The weight ratio between the silica component and the starch component of the composite particles was 15/85.

### [Example 26]

In the present example, 250 g of the "powder of the starch having a reduced globulin content" prepared in Example 10, instead of the MOCHIRU B in Example 12, was suspended in 4750 g of pure water. Thereafter, composite particles were prepared in a similar manner to Example 12. The globulin residual amount of the composite particles obtained in the present example was 0.01% by weight.

### [Example 27]

In the present example, 250 g of the "powder of the starch having a reduced globulin content" prepared in Example 11, instead of the MOCHIRU B in Example 12, was suspended in 4750 g of pure water. Thereafter, composite particles were prepared in a similar manner to Example 12. The globulin residual amount of the composite particles obtained in the present example was 0.01% by weight.

### [Comparative Example 4]

The same operation as in Example 12 was performed, except that the dehydration conditions of the emulsified liquid were changed to 45°C for 3 hours. However, the substance obtained by performing filtration and washing after dehydration had a film shape, and particles could not be observed in the observation through an optical microscope. It is considered that dehydration was insufficient, and this caused coalescence of liquid droplets, with the result that particles could not be prepared.

### [Comparative Example 5]

As starch kernels, a Fine Snow manufactured by Joetsu Starch Co., Ltd. was used. Otherwise, the procedure is similar to that of Example 12.

### [Comparative Example 6]

In the present comparative example, a dispersion liquid A having a solid content concentration of 20% by weight, obtained by suspending 1000 g of a MOCHIRU B to 4000 g of pure water, was used instead of the dispersion liquid A in Example 12. To this dispersion liquid A, 3333 g of a silica sol (Cataloid SI-30) manufactured by JGC Catalysts and Chemicals Ltd. was added to prepare a dispersion liquid B having a solid content concentration of 24% by weight. Thereafter, the procedure is similar to that of Example 16.

### [Comparative Example 7]

To the dispersion liquid A (5000 g) prepared in Comparative Example 6, 175 g of a silica sol (Cataloid SI-30) manufactured by JGC Catalysts and Chemicals Ltd. was added to prepare a dispersion liquid B having a solid content concentration of 20% by weight. The silica component in solid content of this dispersion liquid B is 5% by weight, and the starch component is 95% by weight. Otherwise, the procedure is the same as in Comparative Example 6.

**[Table 5]**

| | spersion liquid A of star | | Silica component | | Dispersion liquid B | | | Emulsification conditions | | | Dehydration (or freezing) conditions | |
|---|---|---|---|---|---|---|---|---|---|---|---|---|
| | **Type of starch** | **Concentration [%]** | **Type of silica component** | **Concentration [%]** | **Silica component [%]** | **Starch component [%]** | **Concentration [%]** | **Dilution concentration of dispersion liquid B [%]** | **Emulsification dispersion rate [rpm]** | **Emulsification time [min.]** | **Condition** | **Time [Hr.]** |
| **Example 12** | **[1]** | **5** | **(1)** | **30** | **50** | **50** | **9** | **1** | **10000** | **10** | **Heating; 60°C** | **16** |
| **Example 13** | **[2]** | **5** | **(1)** | **30** | **50** | **50** | **9** | **1** | **10000** | **10** | **Heating; 60°C** | **16** |
| **Example 14** | **[1]** | **5** | **(1)** | **30** | **50** | **50** | **9** | **9** | **2000** | **10** | **Heating; 60°C** | **24** |
| **Example 15** | **[1]** | **5** | **(1)** | **30** | **50** | **50** | **9** | **9** | **5000** | **10** | **Heating; 60°C** | **16** |
| **Example 16** | **[1]** | **5** | **(1)** | **30** | **50** | **50** | **9** | **9** | **800** | **10** | **Heating; 60°C** | **24** |
| **Example 17** | **[1]+[3]** | **5** | **(1)** | **30** | **50** | **50** | **9** | **1** | **10000** | **10** | **Heating; 60°C** | **16** |
| **Example 18** | **[1]+[3]** | **5** | **(1)** | **30** | **50** | **50** | **9** | **1** | **10000** | **10** | **Heating; 60°C** | **16** |
| **Example 19** | **[1]** | **5** | **(1)** | **30** | **50** | **50** | **9** | **9** | **10000** | **10** | **Freezing; -25°C** | **72** |
| **Example 20** | **[1]** | **5** | **(2)** | **20** | **50** | **50** | **8** | **1** | **10000** | **10** | **Heating; 60°C** | **16** |
| **Example 21** | **[1]** | **5** | **(3)** | **16** | **50** | **50** | **8** | **1** | **10000** | **10** | **Heating; 60°C** | **16** |
| **Example 22** | **[1]** | **5** | **(1)** | **30** | **25** | **75** | **6** | **1** | **10000** | **10** | **Heating; 60°C** | **16** |
| **Example 23** | **[1]** | **5** | **(1)** | **30** | **15** | **85** | **6** | **1** | **10000** | **10** | **Heating; 60°C** | **16** |
| **Example 24** | **[1]** | **5** | **(4)** | **5** | **50** | **50** | **5** | **1** | **10000** | **10** | **Heating; 60°C** | **16** |
| **Example 25** | **[1]** | **10** | **(4)** | **4.5** | **The surface of starch particles (nucleus particles) is coated with a silica layer by a coating method.** | | | | | | | |
| **Example 26** | **A** | **5** | **(1)** | **30** | **50** | **50** | **9** | **1** | **10000** | **10** | **Heating; 60°C** | **16** |
| **Example 27** | **B** | **5** | **(1)** | **30** | **50** | **50** | **9** | **1** | **10000** | **10** | **Heating; 60°C** | **16** |
| **Comparative Example 4** | **[1]** | **5** | **(1)** | **30** | **50** | **50** | **9** | **1** | **10000** | **10** | **Heating; 45°C** | **3** |
| **Comparative Example 5** | **[3]** | **5** | **(1)** | **30** | **50** | **50** | **9** | **1** | **10000** | **10** | **Heating; 60°C** | **16** |
| **Comparative Example 6** | **[1]** | **20** | **(1)** | **30** | **50** | **50** | **24** | **24** | **800** | **10** | **Heating; 60°C** | **24** |
| **Comparative Example 7** | **[1]** | **20** | **(1)** | **30** | **5** | **95** | **20** | **20** | **800** | **10** | **Heating; 60°C** | **24** |

| | | | | | | | | | | | | |
|---|---|---|---|---|---|---|---|---|---|---|---|---|
| [1]: MOCHIRU B (amylopectin 100%, derived from glutinous rice) manufactured by Joetsu Starch Co., Ltd. [2]: Waxy Starch Y (amylopectin 100%, derived from corn) manufactured by Sanwa Starch Co., Ltd. [3]: Fine Snow (amylopectin 80%, derived from nonglutinous rice) manufactured by Joetsu Starch Co., Ltd. A: Powder having a globulin residual amount lower than [1] B: Powder having a globulin residual amount lower than "[1] + [3]" (1): Cataloid SI-30 (average particle diameter 11 nm, solid content concentration 30% by weight) manufactured by JGC Catalysts and Chemicals Ltd. (2): Cataloid SI-550 (average particle diameter 5 nm, solid content concentration 20% by weight) manufactured by JGC Catlysts and Chemicals Ltd. (3): SS-160 (average particle diameter 160 nm, solid content concentration 16% by weight) manufactured by JGC Catalysts and Chemicals Ltd. (4): Silicic acid solution | | | | | | | | | | | | |

**[Table 6]**

| | **Composite particles** | | | | | | | | | | | |
|---|---|---|---|---|---|---|---|---|---|---|---|---|
| | **Silica component (%)** | **Starch component (%)** | **Amylopectin amount (%) in starch component** | **Average particle diameter (d₁) µm.** | **Maximum particle diameter (d₂) µm** | **d₂/d₁** | **Sphericity** | **Coefficient of variance of particles (CV) %** | **Pore volume ml/g** | **d₃/d₁** | **V₂/V₁** | **Viscosity ratio before and after heating** |
| **Example 12** | **50** | **50** | **100** | **1.4** | **3.4** | **2.4** | **0.95** | **28** | **0.2** | **1.01** | **1.2** | **1.4** |
| **Example 13** | **50** | **50** | **100** | **1.5** | **3.4** | **2.3** | **0.95** | **29** | **0.2** | **1.01** | **1.2** | **1.4** |
| **Example 14** | **50** | **50** | **100** | **11.3** | **29.9** | **2.6** | **0.89** | **39** | **0.2** | **1.01** | **1.1** | **1.3** |
| **Example 15** | **50** | **50** | **100** | **8.2** | **19.9** | **2.4** | **0.90** | **37** | **0.2** | **1.01** | **1.1** | **1.3** |
| **Example 16** | **50** | **50** | **100** | **17.7** | **29.9** | **1.7** | **0.90** | **41** | **0.2** | **1.00** | **1.3** | **1.3** |
| **Example 17** | **50** | **50** | **90** | **1.7** | **4.5** | **2.6** | **0.93** | **33** | **0.2** | **1.03** | **1.5** | **1.8** |
| **Example 18** | **50** | **50** | **95** | **1.5** | **4.5** | **3.0** | **0.93** | **32** | **0.2** | **1.02** | **1.3** | **1.7** |
| **Example 19** | **50** | **50** | **100** | **8.8** | **22.8** | **2.6** | **0.86** | **34** | **0.7** | **1.01** | **1.4** | **1.4** |
| **Exanipl e 20** | **50** | **50** | **100** | **1.2** | **3.4** | **2.8** | **0.95** | **30** | **0.2** | **1.01** | **1.1** | **1.4** |
| **Exanple21** | **50** | **50** | **100** | **1.6** | **3.4** | **2.1** | **0.93** | **31** | **0.3** | **1.01** | **1.3** | **1.4** |
| **Example 22** | **25** | **75** | **100** | **1.6** | **3.4** | **2.1** | **0.91** | **32** | **0.2** | **1.01** | **1.5** | **1.6** |
| **Exanple23** | **15** | **85** | **100** | **1.8** | **3.4** | **1.9** | **0.91** | **33** | **0.2** | **1.02** | **1.7** | **1.7** |
| **Example 24** | **50** | **50** | **100** | **1.5** | **3.4** | **2.3** | **0.95** | **28** | **0.1** | **1.01** | **1.1** | **1.4** |
| **Example 25** | **15** | **85** | **100** | **7.0** | **17.4** | **2.5** | **0.85** | **35** | **0.0** | **1.01** | **1.1** | **1.6** |
| **Example 26** | **50** | **50** | **100** | **1.5** | **3.6** | **2.4** | **0.95** | **28** | **0.2** | **1.01** | **1.2** | **1.4** |
| **Example 27** | **50** | **50** | **90** | **1.9** | **5.2** | **2.7** | **0.93** | **33** | **0.2** | **1.03** | **1.5** | **1.7** |
| **Comparative Example 4** | **50** | **50** | **100** | **-** | **-** | **-** | **-** | **-** | **-** | **-** | **-** | **-** |
| **Comparative Example 5** | **50** | **50** | **80** | **1.5** | **4.5** | **3.0** | **0.88** | **33** | **0.1** | **1.30** | **2.3** | **2.3** |
| **Comparative Example 6** | **50** | **50** | **100** | **25.3** | **101.5** | **4.0** | **0.84** | **58** | **0.2** | **1.02** | **1.1** | **1.1** |
| **Comparative Example 7** | **5** | **95** | **100** | **29.0** | **101.5** | **3.5** | **0.85** | **55** | **0.2** | **1.20** | **5.5** | **5.5** |

### [Touch Properties of Powder of Particles]

Next, touch properties of the powders obtained in Examples and Comparative Examples were evaluated. The powders were each subjected to a sensory test by 20 professional panelists. The panelists were interviewed regarding seven evaluation items: smooth and dry feel, moist feel, rolling feel, uniform spreadability, adhesiveness to skin, persistence of rolling feel, and soft feel. The evaluation points by the panelists based on evaluation point criteria (a) were summed, and touch properties were evaluated based on evaluation criteria (b). The results are illustrated in Table 7. As a result, it was found that the powders of Examples are significantly excellent as a touch improver of cosmetics, but the powders of Comparative Examples are not suitable as a touch improver.

### Evaluation point criteria (a)

5 points: very good
4 points: good
3 points: average
2 points: poor
1 point: very poor

### Evaluation criteria (b)

Excellent: not less than 80 points in total
Good: not less than 60 points and less than 80 points in total
Fair: not less than 40 points and less than 60 points in total
Poor: not less than 20 points and less than 40 points in total
Bad: less than 20 points in total

**[Table 7]**

| Evaluation sample | Smooth and dry feel | Moist feel | Rolling feel | Uniform spread ability | Adhesiveness to skin | Persistence of rolling feel | Soft feel |
|---|---|---|---|---|---|---|---|
| Example 1 | Good | Excellent | Good | Good | Excellent | Good | Excellent |
| Example 2 | Excellent | Good | Good | Fair | Good | Good | Good |
| Example 3 | Excellent | Excellent | Excellent | Good | Good | Excellent | Excellent |
| Example 4 | Good | Excellent | Good | Good | Excellent | Good | Excellent |
| Example 5 | Excellent | Fair | Excellent | Poor | Fair | Excellent | Fair |
| Example 6 | Good | Good | Good | Good | Good | Good | Good |
| Example 7 | Excellent | Fair | Excellent | Fair | Fair | Excellent | Fair |
| Example 8 | Good | Good | Good | Good | Good | Good | Excellent |
| Example 9 | Good | Excellent | Good | Good | Good | Good | Excellent |
| Example 10 | Good | Excellent | Good | Good | Excellent | Good | Excellent |
| Example 11 | Good | Good | Good | Good | Good | Good | Excellent |
| Comparative Example 1 | - | - | - | - | - | - | - |
| Comparative Example 2 | Bad | Excellent | Bad | Bad | Excellent | Bad | Good |
| Comparative Example 3 | Excellent | Bad | Excellent | Bad | Bad | Excellent | Bad |
| Example 12 | Good | Good | Good | Good | Good | Good | Good |
| Example 13 | Good | Good | Good | Good | Good | Good | Good |
| Example 14 | Good | Fair | Excellent | Good | Fair | Good | Fair |
| Example 15 | Good | Good | Excellent | Excellent | Good | Good | Good |
| Example 16 | Excellent | Poor | Excellent | Poor | Poor | Good | Poor |
| Example 17 | Good | Good | Good | Good | Good | Poor | Good |
| Example 18 | Good | Good | Good | Good | Good | Fair | Good |
| Example 19 | Good | Poor | Good | Good | Good | Poor | Excellent |
| Example 20 | Excellent | Good | Good | Excellent | Good | Good | Good |
| Example 21 | Poor | Excellent | Good | Fair | Good | Good | Good |
| Example 22 | Fair | Good | Good | Good | Good | Good | Excellent |
| Example 23 | Fair | Excellent | Good | Good | Good | Good | Excellent |
| Example 24 | Good | Excellent | Good | Good | Good | Good | Good |
| Example 25 | Good | Fair | Good | Good | Good | Fair | Excellent |
| Example 26 | Good | Good | Good | Good | Good | Good | Good |
| Example 27 | Good | Good | Good | Good | Good | Poor | Good |
| Comparative Example 4 | - | - | - | - | - | - | - |
| Comparative Example 5 | Bad | Excellent | Bad | Bad | Excellent | Bad | Good |
| Comparative Example 6 | Excellent | Bad | Excellent | Bad | Bad | Excellent | Bad |
| Comparative Example 7 | Bad | Excellent | Bad | Bad | Bad | Bad | Excellent |

### [Usage Feel of Powder Foundation]

With the powder of the composite particles, a powder foundation was prepared in accordance with the formulation ratio (% by weight) illustrated in Table 8. That is, a component (1) as the powder of each example was poured together with components (2) to (9) in a mixer, and these were stirred so as to be uniformly mixed. Next, cosmetic components (10) to (12) were poured in this mixer, and these were stirred again so as to be uniformly mixed. The obtained cake-like substance was crushed. Thereafter, about 12 g of the substance was taken out from the crushed substance, and then put in a rectangular metal dish of 46 mm × 54 mm × 4 mm, and press-molded. The thus obtained powder foundation was subjected to a sensory test by 20 professional panelists. The panelists were interviewed regarding six evaluation items: uniform spread, moist feel, and smoothness during application on skin, and uniformity of a cosmetic film, moist feel, and softness after application on skin. The evaluation points by the panelists based on the above-described evaluation point criteria (a) were summed, and the usage feel of the foundation was evaluated based on the above-described evaluation criteria (b). The results are illustrated in Table 9. The cosmetics according to Examples have an excellent usage feel both during and after the application. However, the cosmetics according to Comparative Examples have a poor usage feel.

**[Table 8]**

| | **Cosmetic ingredients constituting powder foundation** | **Blend ratio [weight (%)]** |
|---|---|---|
| **(1)** | **Powder according to Example or Comparative Example** | **10.0** |
| **(2)** | **Sericite (silicon treatment)** | **40.0** |
| **(3)** | **Talc (silicon treatment)** | **29.0** |
| **(4)** | **Mica (silicon treatment)** | **5.0** |
| **(5)** | **Titanium oxide (silicon treatment)** | **7.0** |
| **(6)** | **Yellow iron oxide (silicon treatment)** | **1.2** |
| **(7)** | **Red iron oxide (silicon treatment)** | **0.4** |
| **(8)** | **Black iron oxide (silicon treatment)** | **0.2** |
| **(9)** | **Methyl paraben** | **0.2** |
| **(10)** | **Dimethicone** | **4.0** |
| **(11)** | **Liquid paraffin** | **2.0** |
| **(12)** | **Glyceryl tri 2-ethylhxanoate** | **1.0** |

**[Table 9]**

| **Evaluation sample** | **During application** | | | **After application** | | |
|---|---|---|---|---|---|---|
| | **Uniform spreadability** | **Moist feel** | **Smoothness** | **Uniformity of film** | **Moist feel** | **Softness** |
| **Example 1 (Cosmetic 1)** | **Good** | **Excellent** | **Excellent** | **Good** | **Excellent** | **Good** |
| **Example 2 (Cosmetic 2)** | **Good** | **Good** | **Good** | **Good** | **Good** | **Excellent** |
| **Example 3 (Cosmetic 3)** | **Excellent** | **Good** | **Good** | **Good** | **Good** | **Excellent** |
| **Example 4 (Cosmetic 4)** | **Good** | **Excellent** | **Excellent** | **Good** | **Excellent** | **Good** |
| **Example 5 (Cosmetic 5)** | **Good** | **Good** | **Good** | **Good** | **Good** | **Good** |
| **Example 6 (Cosmetic 6)** | **Excellent** | **Fair** | **Fair** | **Excellent** | **Fair** | **Good** |
| **Example 7 (Cosmetic 7)** | **Excellent** | **Poor** | **Good** | **Good** | **Poor** | **Fair** |
| **Example 8 (Cosmetic 8)** | **Good** | **Good** | **Good** | **Good** | **Good** | **Good** |
| **Example 9 (Cosmetic** 9) | **Good** | **Excellent** | **Good** | **Good** | **Good** | **Good** |
| **Example 10 (Cosmetic 10)** | **Good** | **Excellent** | **Excellent** | **Good** | **Excellent** | **Good** |
| **Example 11 (Cosmetic 11)** | **Good** | **Good** | **Good** | **Good** | **Good** | **Good** |
| **Comparative Example 1 (Cosmetic a)** | **-** | **-** | **-** | **-** | **-** | **-** |
| **Comparative Example 2 (Cosmetic b)** | **Bad** | **Excellent** | **Poor** | **Bad** | **Excellent** | **Bad** |
| **Comparative Example 3 (Cosmetic c)** | **Bad** | **Poor** | **Bad** | **Bad** | **Poor** | **Poor** |
| **Example 12 (Cosmetic 12)** | **Good** | **Good** | **Good** | **Good** | **Good** | **Good** |
| **Example 13 (Cosmetic 13)** | **Good** | **Good** | **Good** | **Good** | **Good** | **Good** |
| **Example 14 (Cosmetic 14)** | **Good** | **Fair** | **Poor** | **Fair** | **Fair** | **Good** |
| **Example 15 (Cosmetic 15)** | **Excellent** | **Good** | **Good** | **Excellent** | **Good** | **Good** |
| **Example 16 (Cosmetic 16)** | **Poor** | **Poor** | **Poor** | **Poor** | **Poor** | **Fair** |
| **Example 17 (Cosmetic 17)** | **Good** | **Good** | **Good** | **Fair** | **Good** | **Good** |
| **Example 18 (Cosmetic 18)** | **Good** | **Good** | **Good** | **Fair** | **Good** | **Good** |
| **Example 19 (Cosmetic 19)** | **Good** | **Poor** | **Good** | **Good** | **Poor** | **Good** |
| **Example 20 (Cosmetic 20)** | **Excellent** | **Good** | **Good** | **Excellent** | **Good** | **Good** |
| **Example 21 (Cosmetic 21)** | **Fair** | **Good** | **Good** | **Fair** | **Good** | **Good** |
| **Example 22 (Cosmetic 22)** | **Good** | **Good** | **Good** | **Good** | **Good** | **Excellent** |
| **Example 23 (Cosmetic 23)** | **Good** | **Excellent** | **Good** | **Good** | **Good** | **Excellent** |
| **Example 24 (Cosmetic 24)** | **Good** | **Good** | **Good** | **Good** | **Excellent** | **Good** |
| **Example 25 (Cosmetic 25)** | **Good** | **Fair** | **Fair** | **Good** | **Good** | **Excellent** |
| **Example 26 (Cosmetic 26)** | **Good** | **Good** | **Good** | **Good** | **Good** | **Good** |
| **Example 27 (Cosmetic 27)** | **Good** | **Good** | **Good** | **Fair** | **Good** | **Good** |
| **Comparative Example 4 (Cosmetic d)** | **-** | **-** | **-** | **-** | **-** | **-** |
| **Comparative Example 5 (Cosmetic e)** | **Bad** | **Excellent** | **Poor** | **Bad** | **Excellent** | **Bad** |
| **Comparative Example 6 (Cosmetic f)** | **Bad** | **Poor** | **Bad** | **Bad** | **Poor** | **Poor** |
| **Comparative Example 7 (Cosmetic g)** | **Bad** | **Bad** | **Bad** | **Bad** | **Bad** | **Excellent** |

## Claims

1. Particles comprising a starch having an amylopectin content of 90% by weight or more, wherein
an average particle diameter d₁ is 0.5 to 20 µm, and
a maximum particle diameter d₂ is less than 30 µm while being less than 3.0 times the average particle diameter.

2. The particles according to claim 1,
wherein the particles contain an inorganic oxide.

3. The particles according to claim 2,
wherein the particles contain the starch in an amount ranging from 30 to 90% by weight and the inorganic oxide in an amount ranging from 10 to 70% by weight.

4. The particles according to claim 1,
wherein the particles are starch particles constituted by starch.

5. The particles according to claim 4,
wherein a specific surface area is 20 m²/g or more.

6. The particles according to claim 4 or 5,
wherein in an aqueous dispersion liquid having a solid content concentration of 50% by weight of the particles, a gelatinization onset temperature based on a DSC curve obtained using a differential scanning calorimeter is 45°C or higher.

7. The particles according to any one of claims 4 to 6,
wherein the particles are hollow particles having a cavity inside a shell.

8. The particles according to claim 2 or 3,
wherein when an aqueous dispersion liquid having a solid content concentration of 10% by weight of the particles is heated at 80°C for 24 hours, a ratio (V₂/V₁) between a viscosity V₂ of the aqueous dispersion liquid after the heating and a viscosity V₁ of the aqueous dispersion liquid before the heating is 2.0 or less.

9. The particles according to any one of claims 1 to 8,
wherein a globulin content is 0.10% by weight or less.

10. The particles according to any one of claims 1 to 9,
wherein a sphericity is 0.85 or more.

11. The particles according to any one of claims 1 to 10,
wherein a coefficient of variance of particles is 50% or less.

12. The particles according to any one of claims 1 to 11,
wherein when the aqueous dispersion liquid of the particles is applied with ultrasonic waves by an ultrasonic disperser for 60 minutes, a ratio (d₃/d₁) between an average particle diameter d₃ after the application and an average particle diameter d₁ before the application is in a range of 0.95 to 1.05.

13. A production method of particles, comprising:
an emulsification step of mixing a dispersion liquid of a starch having an amylopectin content of 90% by weight or more, a surfactant, and a nonaqueous solvent to prepare an emulsified liquid containing emulsified droplets;
a dehydration step of removing water from the emulsified droplets; and
a step of separating the nonaqueous solvent dispersion body obtained in the dehydration step into solid and liquid to obtain starch particles as solid matter.

14. The production method of particles according to claim 7,
wherein the dehydration step is performed after cooling the emulsified liquid obtained in the emulsification step to a range of -50°C to 0°C to prepare a frozen emulsified liquid in which water in the emulsified droplets is frozen and then returning the frozen emulsified liquid to normal temperature.

15. Cosmetics in which the particles according to any one of claims 1 to 12 are formulated.
